(19) Europäisches Patentamt / European Patent Office / Office européen des brevets

(11) **EP 4 212 242 A1**

(12) **EUROPEAN PATENT APPLICATION**
published in accordance with Art. 153(4) EPC

(43) Date of publication:
**19.07.2023 Bulletin 2023/29**

(21) Application number: **21866649.3**

(22) Date of filing: **02.09.2021**

(51) International Patent Classification (IPC):
**B01J 20/14** (2006.01)   **A61L 9/01** (2006.01)
**A61L 9/014** (2006.01)   **B01J 2/28** (2006.01)
**B01J 20/28** (2006.01)   **B01J 20/32** (2006.01)

(52) Cooperative Patent Classification (CPC):
**A61L 9/01; A61L 9/014; B01J 2/28; B01J 20/14; B01J 20/28; B01J 20/32**

(86) International application number:
**PCT/JP2021/032310**

(87) International publication number:
**WO 2022/054690 (17.03.2022 Gazette 2022/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.09.2020 JP 2020153950**

(71) Applicants:
• **Daikin Industries, Ltd.**
**Osaka-shi, Osaka 530-0001 (JP)**
• **The University of Tokyo**
**Bunkyo-ku, Tokyo 113-8654 (JP)**

(72) Inventors:
• **NISHIMURA, Mayu**
**Osaka-shi, Osaka 5300001 (JP)**
• **MATSUOKA, Takuya**
**Osaka-shi, Osaka 5300001 (JP)**
• **SAKATA, Ichiro**
**Tokyo 113-8654 (JP)**
• **FUGETSU, Bunshi**
**Tokyo 113-8654 (JP)**
• **UEKI, Takayuki**
**Tokyo 113-8654 (JP)**

(74) Representative: **Hoffmann Eitle**
**Patent- und Rechtsanwälte PartmbB**
**Arabellastraße 30**
**81925 München (DE)**

(54) **ADSORBENT AND GRANULATED SUBSTANCE**

(57) An adsorbent and a granulated substance for which reduction in adsorption performance in low humidity environments is suppressed are provided. The adsorbent includes: a porous body mainly composed of silicon dioxide, including a plurality of fine pores, and having a specific surface area of not less than 1 $m^2/g$ and not more than 10 $m^2/g$; one of an acid and a base with which inside of the fine pores of the porous body is impregnated to neutralize a target gas to generate a salt; and a hydrophilic fiber held in the porous body.

FIG.1

EP 4 212 242 A1

**Description**

Technical Field

The present disclosure relates to an adsorbent and a granulated substance.

Background Art

Patent Document 1 describes an adsorbent composed mainly of diatomaceous earth with acids added.

Citation List

Patent Literature

Patent Document 1: Japanese Patent Laid-Open No. 2009-125668

Summary of the Invention

Technical Problem

Adsorbents and granulated substances having porous bodies such as diatomaceous earth and acids or bases that neutralize gas to be adsorbed sometimes reduce adsorption efficiency of the gas to be adsorbed in low humidity environments.

An object of the present disclosure is to provide an adsorbent and a granulated substance with suppressed reduction in adsorption performance in low humidity environments.

Solution to Problem

An adsorbent according to the present disclosure includes: a porous body mainly composed of silicon dioxide, including plural fine pores, and having a specific surface area of not less than 1 $m^2$/g and not more than 10 $m^2$/g; one of an acid and a base with which inside of the fine pores of the porous body is impregnated to neutralize a target gas to generate a salt; and a hydrophilic fiber held in the porous body.

Here, the porous body may be diatomaceous earth.

In addition, a fiber diameter of the hydrophilic fiber may be less than 1 $\mu$m.

In addition, the hydrophilic fiber may contain a cellulose-based fiber.

In addition, the cellulose-based fiber may contain a cellulose nanofiber.

In addition, polyphosphoric acid may be contained as the acid.

From another perspective, a granulated substance according to the present disclosure is made of the adsorbent.

Here, carboxymethylcellulose may be contained as a binder.

In addition, a content of the carboxymethylcellulose may be in a range of not less than 0.5 wt% and not more than 1.5 wt%.

Advantageous Effects of Invention

According to the present disclosure, it is possible to provide an adsorbent and a granulated substance with suppressed reduction in adsorption performance in low humidity environments.

Brief Description of Drawings

FIG. 1 is a graph showing a relationship between humidity and the amount of adsorption of ammonia for granulated substances in each of Example 1 and Comparative example 1;
FIG. 2 is a graph showing a relationship between adsorption efficiency of ammonia and elapsed time by adsorbents in each of Examples 2 and 3; and
FIG. 3 is a graph showing a relationship between the content of carboxymethylcellulose and the amount of adsorption of ammonia for granulated substances in Example 4.

Description of Embodiment

Hereinafter, an embodiment will be described in detail.

<Adsorbent>

The adsorbent of the present embodiment contains porous bodies, acids or bases, and hydrophilic fibers. In addition, the adsorbent may contain additives other than the porous bodies, acids or bases, and hydrophilic fibers, as necessary.

In the adsorbent, plural pores formed in the porous body are impregnated with the acids or bases. In addition, in the adsorbent, the hydrophilic fibers are held in the porous body.

The adsorbent in the present embodiment adsorbs gas. Though the details will be described later, in the adsorbent, the acids or bases neutralize the gas to be adsorbed by the adsorbent, to thereby generate salts. As a result, the gas to be adsorbed by the adsorbent is decomposed. In addition, in the case where the gas to be adsorbed by the adsorbent has an odor, the odor is removed by decomposing the gas.

Subsequently, each component constituting the adsorbent in the present embodiment will be sequentially described.

(Porous body)

In the adsorbent of the present embodiment, a porous body, which is mainly composed of silicon dioxide, includes plural fine pores, and has a specific surface area of 1 $m^2$/g or more and 10 $m^2$/g or less, can be used. Note that the specific surface area of the porous body can be measured by the gas adsorption method using nitrogen gas, for example.

Specific examples of such porous bodies include diatomaceous earth, silica gel, and mesoporous silica, and it is preferable to use diatomaceous earth.

(Diatomaceous earth)

Diatomaceous earth is made of soil mainly formed by accumulation of diatom frustules, and is mainly composed of silicon dioxide. There are marine and freshwater species of diatomaceous earth, both of which can be used.

Diatomaceous earth has a porous structure including plural pores communicating with one another. Some of the pores formed in diatomaceous earth communicate to the outside of diatomaceous earth. In addition, the pore diameter of plural pores formed in diatomaceous earth falls in the range from about 0.1 $\mu$m to several tens of $\mu$m, though being different depending on the type and origin of diatomaceous earth.

Diatomaceous earth has the porous structure to allow water to be adsorbed inside the pores. This prevents liberation of acids or bases from diatomaceous earth by the water existing around the adsorbent by adsorbing water inside the pores in high humidity environments.

In addition, diatomaceous earth has the porous structure to increase the surface area of diatomaceous earth. This allows diatomaceous earth to hold more acids or bases inside the plural pores. Then, it is possible to facilitate the neutralization reaction between the gas to be adsorbed by the adsorbent and the acids or bases.

(Acid or base)

As described above, the inside of the plural pores formed in the porous body is impregnated with the acids or bases. Note that the acids or bases may exist outside the plural pores formed in the porous body, in addition to the inside of the plural pores. Then, the acids or bases neutralize the gas to be adsorbed by the adsorbent, to thereby form salts.

The acids or bases are selected in accordance with the gas to be adsorbed by the adsorbent. For example, in the case where the gas to be adsorbed by the adsorbent is a basic gas, acids are used. In the case where the gas to be adsorbed by the adsorbent is an acid gas, bases are used. Note that, in the following description, the gas to be adsorbed by the adsorbent is referred to as an adsorption-target gas in some cases.

Acids are not particularly limited as long as the acids neutralize the basic adsorption-target gas to generate salts. Specific examples of such acids include polyphosphoric acid, phosphoric acid, sulfuric acid, nitric acid, oxalic acid, and citric acid, but are not particularly limited thereto. Among these acids, it is preferable to use polyphosphoric acid and phosphoric acid due to ease of impregnation of porous bodies, stability in the adsorbent, costs, and so on, and it is more preferable to use polyphosphoric acid.

In addition, specific examples of basic adsorption-target gas include ammonia, and trimethylamine, but are not particularly limited thereto.

Bases are not particularly limited as long as the bases neutralize the acid adsorption-target gas to generate salts. Specific examples of such bases include sodium hydroxide, potassium hydroxide, sodium carbonate, potassium carbonate, sodium phosphate, and potassium phosphate. Among these bases, it is preferable to use sodium hydroxide and potassium hydroxide due to ease of impregnation of porous bodies, stability in the adsorbent, costs, and so on.

In addition, specific examples of acid adsorption-target gas include hydrogen sulfide, methyl mercaptan, and sulfur dioxide, but are not particularly limited thereto.

(Hydrophilic fiber)

As described above, the hydrophilic fibers are held in the porous body. More specifically, the hydrophilic fibers are held outside of the plural pores formed in the porous body. Note that some of the hydrophilic fibers may exist inside the plural pores formed in the porous body.

Then, the hydrophilic fibers adsorb water around the adsorbent or water generated by the neutralization reaction between the adsorption-target gas and the acids or bases.

Here, in the present embodiment, the "hydrophilic fiber" means a fiber with a polar group that is easy to attract water to the surface thereof. Specific examples of polar groups that are easy to attract water include hydroxy groups (-OH), amino groups (-NH$_2$), carboxy groups (-COOH), ether bonds (-O-), and amide bonds (-CONH-). These polar groups perform hydrogen bond with water, to thereby attract the water to the surface of the fiber.

It is preferable that the fiber diameter of the hydrophilic fiber is less than 1 $\mu$m. Since the fiber diameter of the hydrophilic fiber is less than 1 $\mu$m, the surface area of the hydrophilic fiber is larger than that of the hydrophilic fiber in the case where the fiber diameter of the hydrophilic fiber is 1 $\mu$m or more. This makes it easier for the hydrophilic fibers to adsorb water around the adsorbent or water generated by the neutralization reaction between the adsorption-target gas and the acids or bases. As a result, reduction in adsorption performance by the adsorbent in low humidity environments can be more suppressed.

Note that the fiber diameter of the hydrophilic fiber can be obtained by the following technique, for example. First, an SEM image of the adsorbent is obtained by use of an electron microscope. On this occasion, a magnification that can recognize the fiber diameter of the hydrophilic fiber is selected as the magnification of the electron microscope. Subsequently, a predetermined number of hydrophilic fibers (for example, 100) are randomly selected from among the plural hydrophilic fibers shown in the obtained SEM image, and the fiber diameter of each hydrophilic fiber is measured. Then, by dividing the sum of all measured fiber diameters by the number of measured fibers, the fiber diameter (average fiber diameter) of the hydrophilic fibers in the adsorbent is obtained.

Specific examples of the hydrophilic fibers include cellulose nanofibers, microcellulose, cellulose-based fibers such as rayon, lyocell, cotton, and hemp, protein-based fibers such as silk, and polyvinyl alcohol. One of these hydrophilic fibers may be solely used, or two or more of them may be used in combination. Among these hydrophilic fibers, it is preferable to use cellulose-based fibers.

Here, the cellulose-based fiber is a fiber mainly composed of cellulose and/or a cellulose derivative. Cellulose has six hydroxy groups and four ether bonds in the repeating unit of the molecular chain as polar groups that attract water by strong hydrogen bond. For this reason, the cellulose-based fibers are highly hydrophilic and easily attract water.

Further, among these cellulose-based fibers, it is more preferable to use cellulose nanofibers. By using cellulose nanofibers as the hydrophilic fibers, the amount of adsorption of gas to be adsorbed by the adsorbent can be increased as compared to the case where other cellulose-based fibers are used.

Here, the cellulose nanofiber refers to a fiber made of cellulose with a fiber diameter of the order of between 1 nm and several hundred nm. In the present embodiment, it is preferable that the fiber diameter of cellulose nanofiber falls in the range from 1 nm or more and 400 nm or less. Cellulose nanofibers are formed, for example, by defibration of pulp fibers with a publicly known method.

(Manufacturing method of adsorbent)

Subsequently, a specific example of the manufacturing method of the adsorbent in the present embodiment will be described. The adsorbent of the present embodiment can be manufactured as follows, for example.

First, at a predetermined weight ratio, the above-described hydrophilic fibers and acids or bases are mixed. Subsequently, in the obtained mixture, porous bodies of a predetermined weight are added step by step, and stirred. With this, granular adsorbents with porous bodies, acids or bases, and hydrophilic fibers can be obtained.

Note that, as long as the adsorbent of the present embodiment contains the porous bodies, acids or bases, and the hydrophilic fibers, the shape of the adsorbent is not limited to a granule.

(Action by adsorbent)

As described above, the adsorbent of the present embodiment neutralizes the acids or bases and the gas to be adsorbed, to thereby generate salts. Here, the efficiency of the neutralization reaction between the acids or bases in the adsorbent and the gas to be adsorbed differs depending on the humidity around the acids or bases. For example, if the humidity around the acids or bases is excessively low, the efficiency of the neutralization reaction between the acids or bases and the gas to be adsorbed is apt to be reduced.

In contrast thereto, the adsorbent of the present embodiment contains the hydrophilic fibers in addition to the porous bodies and the acids or bases; accordingly, water can be held by the hydrophilic fibers even in the low humidity environments. This prevents the humidity around the acids or bases from extremely decreasing. As a result, in the low humidity environments, the reduction in the efficiency of the neutralization reaction between the acids or bases and the gas to be adsorbed can also be suppressed.

<Granulated substance>

The above-described adsorbent may be formed as a granulated substance by use of publicly known methods. Subsequently, the granulated substance made of the adsorbent will be described.

Here, in the granulated substance containing the porous bodies and the acids or bases neutralizing the gas to be adsorbed, the following problems occur in some cases. In such a granulated substance, adjacent granulated substances sometimes adhere to each other when salts and water are generated by neutralization reaction between the adsorption-target gas and the acids or bases. When the granulated substances adhere to one another, it becomes difficult for the adsorption-target gas to pass between the granulated substances, and there is a risk of reduction in the adsorption performance by the granulated substances.

In contrast thereto, the granulated substance of the present embodiment is configured with the adsorbent containing the hydrophilic fibers in addition to the porous bodies and the acids or bases. This causes part of the water generated by the neutralization reaction between the adsorption-target gas and the acids or bases to be adsorbed to the hydrophilic fibers. As a result, the adhesion of granulated substances to one another is suppressed, and the reduction in the adsorption performance is prevented.

(Binder)

Here, the granulated substance of the present embodiment may further contain binders in addition to the porous bodies, the acids or bases, and the hydrophilic fibers. The inclusion of binders in the granulated substance increases the strength of the granulated substance.

Specific examples of the binder include, though not particularly limited, cellulose derivatives such as methylcellulose, ethylcellulose, propylcellulose, carboxymethylcellulose, carboxymethylcellulose, hydroxymethylcellulose, and hydroxyethylcellulose, polyvinyl alcohol, and acrylic resin. Among these, it is preferable to use binders composed of cellulose derivatives, and it is more preferable to use carboxymethylcellulose. One of these binders may be solely used, or two or more of them may be used in combination.

In the case where carboxymethylcellulose is used as the binder, it is preferable that the content of carboxymethylcellulose in the granulated substance is in a range of 0.5 wt% or more and 1.5 wt% or less. By setting the content of carboxymethylcellulose in the granulated substance to the above range, the amount of adsorption of the gas to be adsorbed can be increased as compared to the case where the content of carboxymethylcellulose exceeds 1.5 wt%, and the adsorption performance by the granulated substance can be increased. In addition, in the case where 0.5 wt% or more of carboxymethylcellulose is contained, it is possible to reduce detachment of powder from the granulated substance and dispersion thereof.

(Manufacturing method of granulated substance)

Subsequently, a specific example of the manufacturing method of the granulated substance in the present embodiment will be described. The granulated substance of the present embodiment can be manufactured as follows, for example.

First, at a predetermined weight ratio, the porous bodies, the acids or bases, the hydrophilic fibers, and the binders as needed are mixed to obtain a mixture. Subsequently, the mixture is formed into pellet shapes by use of a granulating device. Subsequently, obtained pellet-shaped materials are heated and dried at a predetermined temperature using a dryer, and then sifted to obtain granulated substances of predetermined shape and size.

[Examples]

Subsequently, the adsorbent and the granulated substance in the present embodiment will be described in more detail using examples. Note that the adsorbent and the granulated substance are not limited to the following examples.

<Study 1>

First, granulated substances containing hydrophilic fibers in addition to diatomaceous earth, which is a specific example of the porous bodies, acids and binders, and granulated substances without containing the hydrophilic fibers were prepared, and the difference in adsorption performance depending on humidity was studied.

1. Preparation of granulated substances

(Example 1)

Diatomaceous earth, polyphosphoric acid, cellulose nanofiber, and carboxymethylcellulose dissolved in water were placed in a kneader at the ratio shown in Table 1 below, and then mixed to prepare a mixture. Here, the blending amount of carboxymethylcellulose (wt%) is the weight of carboxymethylcellulose, not containing the weight of the water used to dissolve carboxymethylcellulose.
Subsequently, the obtained mixture was introduced into a granulating device (Multigran manufactured by Dalton Corporation), and thereby pellet-shaped materials were obtained. Subsequently, the obtained pellet-shaped materials were dried in a dryer at 100°C for 20 minutes, and then sifted through to obtain granulated substances with a diameter of about 2 mm to about 3 mm.

(Comparative example 1)

Granulated substances were obtained in the same manner as in Example 1 except that cellulose nanofibers, which are hydrophilic fibers, are not contained, and except for the ratio of each material to be mixed.

[Table 1]

|  | Diatomaceous earth (wt%) | Polyphosphoric acid (wt%) | Cellulose nanofiber (wt%) | Carboxymethylcellulose (wt%) |
|---|---|---|---|---|
| Example 1 | 47.3 | 47.3 | 3.4 | 2.0 |
| Comparative example 1 | 49.5 | 49.5 | 0.0 | 1.0 |

2. Adsorption performance test

Subsequently, the adsorption performance of granulated substances obtained in Example 1 and Comparative example 1 was tested for ammonia, which is a basic adsorption-target gas.
Specifically, air with a humidity of 13%RH and an ammonia concentration of 200 ppm was continuously passed into the granulated substances obtained in Example 1 and Comparative example 1 so that the SV (Space Velocity) value was 53,000. The concentration of ammonia in the air upstream and downstream of the granulated substances was measured, and the amount of ammonia adsorbed by the granulated substances was calculated using the difference between the concentration of ammonia in the upstream side and the concentration of ammonia in the downstream side. Note that the amount of ammonia adsorbed by the granulated substances was calculated as the amount of adsorption of ammonia to 1g of polyphosphoric acid contained in the granulated substances (the amount of adsorption of ammonia (mg)/1g of polyphosphoric acid).
In addition, the amount of ammonia adsorbed by the granulated substances (the amount of adsorption of ammonia (mg)/1g of polyphosphoric acid) was calculated for Example 1 and Comparative example 1 in the same manner except for changing the humidity of the air containing ammonia to 25%RH, 45%RH, and 67%RH.

3. Test results

Table 2 shows the measurement results of the amount of adsorption of ammonia for the granulated substances in Example 1 and Comparative example 1 in the cases where the humidity of air containing ammonia was set to 13%RH, 25%RH, 45%RH, and 67%RH.

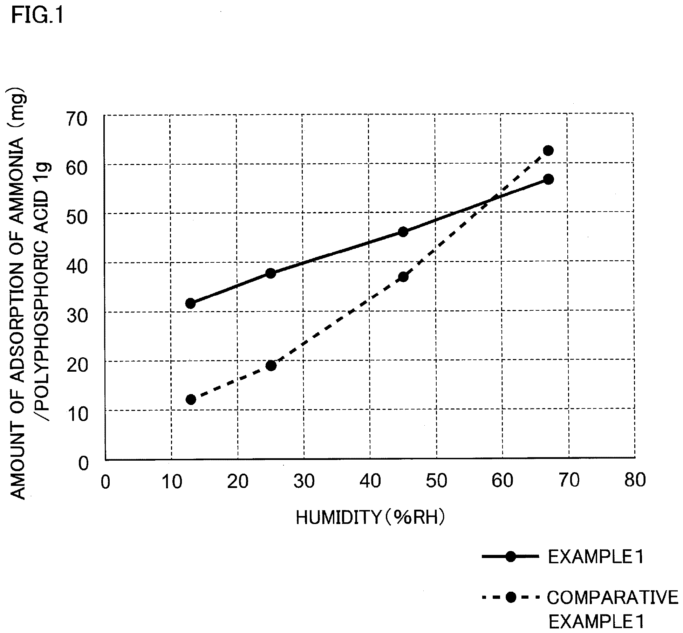

FIG. 1 is a graph showing a relationship between humidity and the amount of adsorption of ammonia for the granulated substances in Example 1 and Comparative example 1.

[Table 2]

| Humidity (%RH) | Amount of adsorption of ammonia (mg)/ 1g of polyphosphoric acid | |
|---|---|---|
| | Example 1 | Comparative example 1 |
| 13 | 32 | 12 |
| 25 | 38 | 19 |
| 45 | 46 | 37 |
| 67 | 57 | 63 |

As shown in Table 2 and FIG. 1, the reduction in the amount of ammonia adsorption in the low humidity environment was suppressed in the granulated substances in Example 1, which contained cellulose nanofibers as the hydrophilic fibers, as compared to the granulated substances in Comparative example 1, which did not contain cellulose nanofibers. Specifically, in the granulated substances in Example 1, the reduction in the amount of adsorption of ammonia was suppressed in the low humidity environment, where the humidity of the air containing ammonia was not more than 50%RH, as compared to the granulated substances in Comparative example 1.

In addition, in the case where the humidity of the air containing ammonia was 67%RH, the amount of adsorption of ammonia was larger in the granulated substances in Comparative example 1 than in the granulated substances in Example 1. However, in the granulated substances in Comparative example 1, when the air containing ammonia was continuously flowing, the granulated substances adhered to one another. As a result, the pressure loss when the air containing ammonia passed through the granulated substances increased. For this reason, it was confirmed that it is difficult to continuously use the granulated substances in Comparative example 1 as an air filter, for example.

On the other hand, in the granulated substances in Example 1, even in the case where the air containing ammonia was continuously passed, the adhesion among the granulated substances was not observed.

<Study 2>

Subsequently, adsorbents with different fiber diameters of hydrophilic fibers were prepared, and the difference in the adsorption performance by the adsorbents was studied.

1. Preparation of adsorbent

(Example 2)

Diatomaceous earth, polyphosphoric acid, and cellulose nanofibers with the average fiber diameter of 50 nm or less were prepared at the ratio shown in Table 3 below. First, polyphosphoric acid and cellulose nanofibers were mixed, and stirred with air using a spatula. Then, diatomaceous earth was added little by little, and each time diatomaceous earth was added, the mixture was stirred with the spatula and the container was shaken, to thereby prepare the granular adsorbents.

(Example 3)

Granular adsorbents were prepared in the same manner as in Example 2 except that microcellulose with the average fiber diameter of 1 μm was used as the hydrophilic fibers instead of cellulose nanofibers, and except for the ratio of each material.

[Table 3]

|  | Diatomaceous earth (wt%) | Polyphosphoric acid (wt%) | Cellulose nanofiber (wt%) | Microcellulose (wt%) |
|---|---|---|---|---|
| Example 2 | 30.6 | 58.9 | 10.5 | 0 |
| Example 3 | 30.0 | 60.0 | 0 | 10.0 |

2. Adsorption performance test

Subsequently, the adsorption performance of adsorbents obtained in Examples 2 and 3 was tested for ammonia, which is a basic adsorption-target gas.

Specifically, similar to Study 1, the air with a humidity of 45%RH and an ammonia concentration of 200 ppm was continuously passed into the adsorbents obtained in Examples 2 and 3, so that the SV value was 53,000. Then, the concentration of ammonia in the air upstream and downstream of the adsorbents was measured. The ammonia concentration was measured at 0 minutes, 15 minutes, 30 minutes, 1 hour, 2 hours, 3 hours, 4 hours, and 5 hours after passing the air containing ammonia through the adsorbents was started. Then, for each elapsed time, the adsorption efficiency of ammonia by the adsorbents was calculated based on the following expression (1).

$$\text{Adsorption efficiency (\%)} = \{1\text{-(ammonia concentration at downstream side/ammonia concentration at upstream side)}\} \times 100 \ ... \ (1)$$

3. Test results

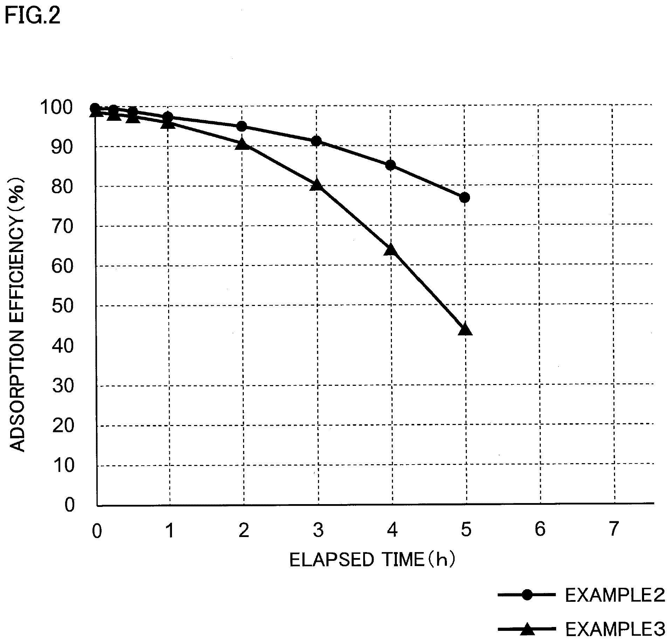

FIG. 2 is a graph showing a relationship between the adsorption efficiency of ammonia and the elapsed time by the adsorbents in Examples 2 and 3.

As shown in FIG. 2, in the adsorbent in Example 2 containing cellulose nanofibers with the fiber diameter less than 1 $\mu$m as the hydrophilic fibers, it took longer to reduce the adsorbent efficiency as compared to the adsorbent in Example 3 containing microcellulose with the fiber diameter of 1 $\mu$m or more. Specifically, the reduction in the adsorption efficiency of the adsorbent in Example 2 to 85% took about 4 hours, whereas the reduction in the adsorption efficiency of the adsorbent in Example 3 to 85% took about 2 hours and 30 minutes.

Consequently, it was confirmed that, due to the fiber diameter of the hydrophilic fiber that was less than 1 $\mu$m, the reduction in the adsorption efficiency by the adsorbent was suppressed and the life of the adsorbent was prolonged as compared to the case where the fiber diameter of the hydrophilic fiber was 1 $\mu$m or more.

In addition, in the adsorbents in Example 2, the amount of adsorption of ammonia per 1 g of polyphosphoric acid was 74 mg when the adsorption efficiency reached 85%. In contrast thereto, in the adsorbents in Example 3, the amount of adsorption of ammonia per 1 g of polyphosphoric acid was 48 mg when the adsorption efficiency reached 85%. Consequently, it was confirmed that, due to the fiber diameter of the hydrophilic fiber that was less than 1 $\mu$m, the amount of adsorption of the gas to be adsorbed by the adsorbents increased as compared to the case where the fiber diameter of the hydrophilic fiber was 1 $\mu$m or more.

<Study 3>

Subsequently, granulated substances with different binder contents were prepared, and the difference in the adsorption performance by the granulated substances was studied.

1. Preparation of granulated substances

(Example 4)

Granulated substances with different contents of carboxycellulose were prepared in the same manner as Example 1 except that the contents of carboxycellulose dissolved in water was set to 1.2 wt%, 3.0 wt%, 4.2 wt%, and 5.0 wt%, and the blending amount of diatomaceous earth and polyphosphoric acid was changed so that the ratio of diatomaceous earth and polyphosphoric acid was 1:1.

2. Adsorption performance test

Subsequently, the adsorption performance of the granulated substances obtained in Example 4 was tested for ammonia, which is a basic adsorption-target gas.

Specifically, similar to Study 1, the air with a humidity of 45%RH and an ammonia concentration of 200 ppm was continuously passed into the granulated substances obtained in Example 4, so that the SV value was 53,000. Then, the concentration of ammonia in the air upstream and downstream of the granulated substances was measured, and the amount of adsorption of ammonia (the amount of adsorption of $NH_3$ (mg)/1g of polyphosphoric acid) was calculated using the difference between the concentration of ammonia in the upstream side and the concentration of ammonia in the downstream side.

3. Test results

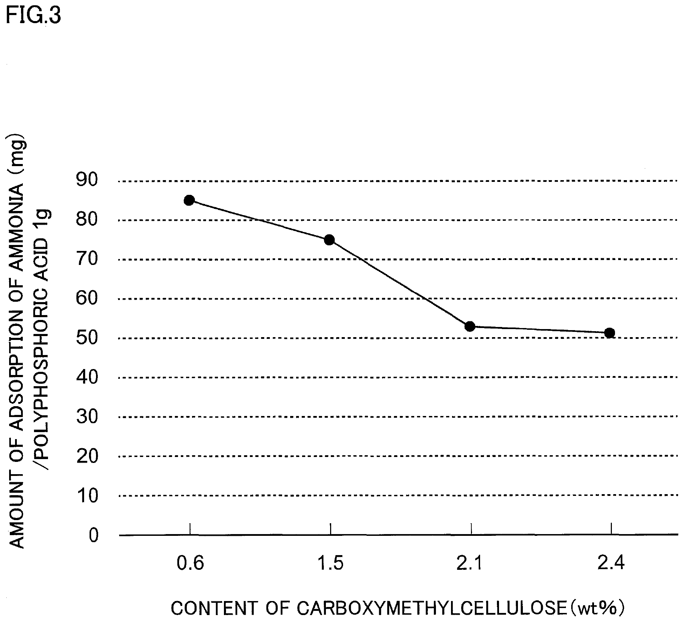

FIG. 3 is a graph showing a relationship between the content of carboxymethylcellulose and the amount of adsorption of ammonia for granulated substances in Example 4.

As shown in FIG. 3, in the granulated substances in Example 4, the amount of adsorption of ammonia was larger in the case where the content of carboxymethylcellulose was 1.5 wt% or less, as compared to the case where the content of carboxymethylcellulose exceeded 1.5 wt%.

Consequently, it was confirmed that, in the case where carboxymethylcellulose was used as the binder in the granulated substances, the content of carboxymethylcellulose was preferably in the range of 0.5 wt% or more and 1.5 wt% or less.

So far the embodiment has been described, and it can be appreciated that various changes in form and detail may be made without departing from the spirit and scope presently or hereafter claimed.

**Claims**

1.   An adsorbent comprising:

    a porous body mainly composed of silicon dioxide, including a plurality of fine pores, and having a specific surface area of not less than 1 $m^2$/g and not more than 10 $m^2$/g;
    one of an acid and a base with which inside of the fine pores of the porous body is impregnated to neutralize a target gas to generate a salt; and
    a hydrophilic fiber held in the porous body.

2.   The adsorbent according to claim 1, wherein the porous body is diatomaceous earth.

3.   The adsorbent according to claim 2, wherein a fiber diameter of the hydrophilic fiber is less than 1 $\mu$m .

4.   The adsorbent according to claim 2, wherein the hydrophilic fiber contains a cellulose-based fiber.

5.   The adsorbent according to claim 4, wherein the cellulose-based fiber contains a cellulose nanofiber.

6.   The adsorbent according to claim 5, wherein polyphosphoric acid is contained as the acid.

7.   A granulated substance comprising:
    the adsorbent according to any one of claims 1 to 6.

8.   The granulated substance according to claim 7, wherein carboxymethylcellulose is contained as a binder.

9.   The granulated substance according to claim 8, wherein a content of the carboxymethylcellulose is in a range of not less than 0.5 wt% and not more than 1.5 wt%.

FIG.1

FIG.2

FIG.3

## INTERNATIONAL SEARCH REPORT

| International application No. |
|---|
| **PCT/JP2021/032310** |

**A. CLASSIFICATION OF SUBJECT MATTER**

*B01J 20/14*(2006.01)i; *A61L 9/01*(2006.01)i; *A61L 9/014*(2006.01)i; *B01J 2/28*(2006.01)i; *B01J 20/28*(2006.01)i; *B01J 20/32*(2006.01)i
FI:   B01J20/14; A61L9/01 B; A61L9/014; B01J2/28; B01J20/28 A; B01J20/32 Z

According to International Patent Classification (IPC) or to both national classification and IPC

**B. FIELDS SEARCHED**

Minimum documentation searched (classification system followed by classification symbols)

B01J2/00-2/30,20/00-20/28,20/30-20/34; A61L9/00-9/22; B01J2/28; B01D53/00-53/90

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2008-200648 A (TORAY IND INC) 04 September 2008 (2008-09-04)<br>claims 1-7, paragraphs [0005]-[0007], [0013]-[0017], [0029]-[0030], [0069]-[0131] | 1-9 |
| A | JP 2008-080328 A (TORAY IND INC) 10 April 2008 (2008-04-10)<br>claims 1-7, paragraphs [0010], [0015], [0040], [0046]-[0048], [0070]-[0137] | 1-9 |
| A | JP 2000-246040 A (MAZDA MOTOR CORP) 12 September 2000 (2000-09-12)<br>entire text, all drawings | 1-2 |
| A | JP 55-024502 A (TAKEDA CHEMICAL INDUSTRIES LTD) 21 February 1980 (1980-02-21)<br>entire text | 1, 6 |
| A | WO 2017/078084 A1 (NIPPON PAPER INDUSTRIES CO., LTD) 11 May 2017 (2017-05-11)<br>paragraphs [0035]-[0037], [0047]-[0072] | 1-5 |
| A | JP 2019-081257 A (KYODO PRINTING CO LTD) 30 May 2019 (2019-05-30)<br>claims 1-9 | 1-9 |

| ✓ | Further documents are listed in the continuation of Box C. | ✓ | See patent family annex. |
|---|---|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **22 October 2021** | **02 November 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

International application No.

**PCT/JP2021/032310**

| C. | DOCUMENTS CONSIDERED TO BE RELEVANT | |
|---|---|---|
| Category* | Citation of document, with indication, where appropriate, of the relevant passages | Relevant to claim No. |
| A | WO 2016/152645 A1 (NITTA CORP) 29 September 2016 (2016-09-29) paragraphs [0039]-[0041], [0064]-[0079], [0087], [0103] | 1, 7-9 |

Form PCT/ISA/210 (second sheet) (January 2015)

International application No.

**PCT/JP2021/032310**

| Patent document cited in search report | | | Publication date (day/month/year) | Patent family member(s) | | | Publication date (day/month/year) |
|---|---|---|---|---|---|---|---|
| JP | 2008-200648 | A | 04 September 2008 | (Family: none) | | | |
| JP | 2008-080328 | A | 10 April 2008 | (Family: none) | | | |
| JP | 2000-246040 | A | 12 September 2000 | US | 6432872 | B1 | |
| | | | | entire text, all drawings | | | |
| | | | | WO | 2000/035580 | A1 | |
| | | | | EP | 1054730 | A1 | |
| | | | | KR | 10-2001-0040870 | A | |
| JP | 55-024502 | A | 21 February 1980 | (Family: none) | | | |
| WO | 2017/078084 | A1 | 11 May 2017 | US | 2018/0318740 | A1 | |
| | | | | paragraphs [0073]-[0121] | | | |
| | | | | EP | 3372618 | A1 | |
| | | | | CN | 108350092 | A | |
| JP | 2019-081257 | A | 30 May 2019 | (Family: none) | | | |
| WO | 2016/152645 | A1 | 29 September 2016 | TW | 201700163 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009125668 A